# EUROPEAN PATENT APPLICATION

(11) **EP 2 898 893 A1**
(43) Date of publication of application: **29.07.2015**
(21) Application number: 14152313.4
(22) Date of filing: 23.01.2014
(51) Int. Cl.: A61K 36/906, A61K 36/9066, A61K 36/9068, A61K 35/413, A61P 1/16

(54) **Improved cholagogue compositions for treating biliary and digestive tract discomfort**

(71) Applicant: Varhalmi, Miklos, West Sussex RH19 4JX (GB)
(72) Inventor: Varhalmi, Miklos, West Sussex RH19 4JX (GB)
(74) Representative: Petho, Arpad

(57) **Abstract**

The invention concerns improved and better ingestible cholagogue compositions possessing improved acceptability in a prolonged treatment regimen and comprising natural substances for treating discomfort associated with biliary and digestive tract diseases and conditions. Methods for producing the compositions of the invention are also disclosed. The active agent of the compositions according to the invention comprises bile liquid extract of animal origin, small amount of turmeric and ginger extract and optionally other active ingredients originating from plants.
The compositions of the invention can be used in treating and alleviating ingestion and biliary related discomfort, gallstone related conditions, certain liver and/or pancreatic complaints, post-biliary surgery complaints. The special combination of components according to the invention facilitates a longer sustainable treatment regimen with the cholagogue compositions of the invention.

## Description

### Field of the invention

The invention concerns improved and better ingestible cholagogue compositions possessing improved acceptability in a prolonged treatment regimen and comprising natural substances for treating discomfort associated with biliary and digestive tract diseases and conditions. Methods for producing the compositions of the invention are also disclosed. The active agent of the compositions according to the invention comprises bile liquid extract of animal origin, small amount of turmeric and ginger extract and optionally other active ingredients originating from plants. The compositions of the invention can be used in treating and alleviating ingestion and biliary related discomfort, gallstone related conditions, certain liver and/or pancreatic complaints, post-biliary surgery complaints. The special combination of components according to the invention facilitates a longer sustainable treatment regimen with the cholagogue compositions of the invention.

### Background of the invention

It is well known in the art that in mammals the liver continuously produces dilute bile liquid, which accumulates in the gallbladder where it is concentrated to 1/10 of its original volume. Bile duct generally opens to the digestive canal at the duodenum. In healthy humans, each time when food enters the duodenum, a valve opens, the gallbladder contracts and discharges its content (the concentrated bile) into the duodenum, so as to allow bowel digestion.

Bile contains bile acids, bile pigments, bilirubin, cholesterol, mucin and lime salts. The absence of bile causes impairment of fat and protein digestion. The hydrotopic bile acids make fatty acids, sterins and other sparingly water-soluble substances easily water-soluble, thereby promoting the absorption of them.

Bile secretion is increased by bile acids, which assure the persistence of bile production by reabsorbing from the intestines. Bile acids play an important role in the defense mechanism of living organisms, as described in László Bertók: "The physico-chemical defense of the living organism", Biológia, 25, 127-134 (1977).

In the case of gallstone and hepatic diseases, abundant bile production is desirable for inhibiting gallstone formation and relieving inflammation of bile ducts, which is facilitated by cholestasis. Cholagogue compositions may also offer a solution for some of the gastrointestinal problems experienced after gallbladder removal, such as bloating or pain.

Other substances increasing the bile production include secretin, histamine; protein breakdown products, peptones and albumoses; volatile oil containing drugs, menthol; radish; podophyllin, mineral water containing Glauber salt; benzoic acid, salicylic acid, aciphenochinoline.

Being dissolved in bile acid, cholesterol is also present in the bile. With secretion of bile certain amount of cholesterol passes out as well. Therefore, blockade of the bile flow increases the level of cholesterol in the blood. High cholesterol level may cause several diseases (vascular lesions).

It is known, that the bile comprises 5 to 6% cholic acid and 0.6 to 0.8% deoxycholic acid bound to glycol and taurine. These cholic acids can be derived from cholanic acid, wherein carbon atoms in positions 3, 7 and 12 may have OH groups.

The bile production enhancing agents, corresponding to the above mentioned ingredients, are usually produced from synthetic substances. The examples of such base materials include dihydroxy cholanic acid, dehydrocholic acid, diketocholanic acid and nicotinic acid hydroxymethylamide. It is disadvantageous that the conformation of synthetically prepared compounds only resembles the molecular structure of substances which can be found in natural bile.

In the pertinent art, there are known approaches wherein substances originating from bile or bile extract have been used for producing medicaments suitable for treating problems of digestion, as well as biliary diseases. Thus, in the patent document No. FR M3607 an oral pharmaceutical composition comprising proteolytic enzyme bromelain and pancreatin enzymes, as well as ox bile extract has been described for compensating the deficient production of digestive enzymes. The three-layer coated tablet formulation produced in accordance with said patent comprises bromelain in the outer gastro-soluble layer, pancreatine in the middle layer which absorbs in the small intestine, and iodochlorohydroxyquinoline and 4,7-phenantroline-5,6-quinone in the inner layer destroying intestinal bacteria. This known formulation compensates for the deficiency in enzyme activity but does not stimulate bile secretion.

Various bilirubin extracts are also known as base materials for medicaments. Several methods are known for producing bilirubin extracts. Thus, in a method described in the published patent document No. CN 87 107 251 (14/09/1988), bilirubin has been extracted from bile of animal origin by organic solvents in the presence of antioxidants. According to another method described in patent document No. CN 1 042 708 (06/06/1990), bilirubin has been extracted from human bile by acid washing at pH 4.5-5.

It is disadvantageous, however, that bilirubin extracts prepared by known methods do not comprise natural bile acids, thereby such extracts are not suitable for enhancing bile secretion.

Because of their side effects, certain synthetic medicaments cannot be used for treating hepatic diseases. Cholagogues generally should not be administered in the case of acute biliary spasm; in this situation the spasm of gallbladder and bile duct smooth muscle should be relaxed, which can be achieved by intravenous administration of various spasmolytics (combined with e.g. morphine in the case of serious pain). Such an injection administered to alleviate frequent bilious attacks can make the patient morphine addicted.

The liver significantly reduces the efficacy and shortens the effect of known medicaments by binding and converting toxins absorbed from intestines, thereby protects the organism from potentially harmful substances. Therefore, in the case of liver function deficiency, such medicaments should be used in smaller amounts, especially during the last term of pregnancy when they can potentially cause foetal damage.

The use of natural products is preferred in chronic medical conditions, e. g. after cholecystectomy or recurrent gallstones as these products usually represent a better tolerable burden to the already precarious health of the gastrointestinal system. Besides, conventional medicine offers limited treatment options. Natural products can also easily be fitted in a holistic approach which is often desired by the nature of the medical condition and also by the patient.

Use of medicinal herb extracts as such or in the form of pharmaceutical formulations or food supplements in the treatment of various medical conditions is well known in the art. Several methods are known in the relevant art for the recovery of active agents as efficiently as possible. Thus, in the patent document No. HU 167 157 a method has been described, wherein the active agent is recovered from drugs of plant origin by extraction with water, organic solvents or a mixture of water and organic solvents in the presence of bile or natural compounds of the bile. Due to the similar function of bile in a living organism, this method provides greater amounts of more efficient active agents than the prior art methods. Extracts recovered in such a way can be used in disinfectants, insecticides and wood impregnation agents, as well as cosmetics.

Patients after gallbladder surgery, patients having gallstones or hepatic disease typically need to take ingestion facilitating agents or dietary supplements for (a) prolonged period(s) and even with such compositions they often suffer gastrointestinal discomfort, e. g. bile reflux or food sensitivities. Medicinal agents and dietary supplements used to treat impaired digestion and especially those of natural origin, comprising bile extracts are often formulated as rather big tablets or capsules and have a distinctive odor that may add up to such discomfort. The tablets cannot be broken into smaller parts because they would be too bitter to swallow and the enterosolvent layer would be damaged. The capsules are designed to be adsorbed only in the intestine thus they must be swallowed whole. Due to the size and odor of the otherwise remedial tablet/capsule a number of patients cease prematurely the recommended regimen, leading to relapse or deterioration of the existing medical condition.

### Summary of the invention

The cholagogue compositions of the present invention suitable for treating discomfort associated with biliary and digestive tract diseases comprise as primary active agent powdered bile liquid extract of animal origin, combined with 1-200%, preferably 1-100%, more preferably 1-10% by weight of an extract produced from turmeric (Curcuma spp.) relative to the weight of the bile extract and 1-100%, preferably 10-50%, more preferably 10-20% by weight of an extract produced from ginger *(Zingiber officinale)* relative to the weight of the bile extract; and optionally, as secondary active agent, 10-20% by weight of papain relative to the weight of the bile extract and/or 5-15% by weight of bromelain relative to the weight of the bile extract and/or 25-75% by weight of α-amylase relative to the weight of the bile extract and/or 25-75% by weight of a lipase of plant origin relative to the weight of the bile extract and/or 70-100% by weight of soy lecithin relative to the weight of the bile extract and/or 20-40% by weight of an extract of milk thistle *(Silybum marianum)* relative to the weight of the bile extract and/or 10-75% by weight of an extract of artichoke *(Cynara scolymus)* relative to the weight of the bile extract and/or 100-150% by weight of an extract of yarrow *(Achillea millefolium)* relative to the weight of the bile extract and/or 5-60% of an extract of peppermint *(Mentha piperita)* relative to the weight of the bile extract and/or 75-120% by weight of an extract of radish *(Raphanus sativus)* relative to the weight of the bile extract and/or 25-40% by weight of an extract of dwarf everlast *(Helichrysum arenarium)* relative to the weight of the bile extract and/or 5-10% by weight of an extract of mallow *(Althea officinalis)* relative to the weight of the bile extract and/or 5-10% by weight of an extract of fennel *(Foeniculum vulgare)* relative to the weight of the bile extract and/or 5-10% by weight of an extract of liquorice *(Glycyrrhiza glabra)* relative to the weight of the bile extract and/or 5-10% by weight of an extract of white horehound *(Marrubium vulgare)* relative to the weight of the bile extract. The improvement of the present compositions over those previously described in Hungarian Patent No. 210 927 lies in the addition of two further components, namely the extracts of turmeric and ginger acting not only as ingestion supporting agents but, highly surprisingly, also as taste and odor masking additives in surprisingly small amounts, making the novel compositions more acceptable to patients and thus enabling the application of a longer and more effective treatment regimen.

### Detailed description of the invention

It was an object of the present invention to further improve the efficacy of the cholagogue compositions previously developed partly by the present inventor and disclosed in Hungarian Patent No. 210 927. I have surprisingly found that when formulated together with two further components, namely the extracts of turmeric and ginger, the ingestion and long term acceptability of the oral cholagogue compositions have significantly been improved. This was highly unexpected considering that the applied amount of either ginger or turmeric alone did not result in any significant taste improvement and although turmeric is a widely used spice, it has a pungent taste and odor when used alone or abundantly. Liquorice, another ingredient with a characteristic taste (sweet) was not effective in masking or improving the distinctive taste and odor of the previous compositions. Apparently, when combined in small quantities, turmeric and ginger have not only enhanced the positive effects of bile and herb extracts on digestion but have also contributed to the improvement of the taste and/or odor of cholagogue compositions comprising thereof making them easier to accept for patients, which effect provides a significant advantage when cholagogue compositions are used in a long term treatment regimen. The increase of compliance leads to less cases of premature cessation of the therapy and a more satisfactory alleviation of the symptoms.

Our previous improvements were based on the finding that if concentrated bile (as primary active agent) is delivered to the duodenum by appropriate oral administration effected shortly after meal, the administered bile is available for bowel digestion, and it ensures the persistence of bile production by reabsorption from the intestines, and, at the same time, the mild purgative effect of the secondary active agents also facilitates the inhibition of gallstone formation and the passing out of biliary sand.

The cholagogues of the present invention are suitable for treating biliary and digestive system conditions and intended for oral administration. They comprise, as primary active agent, powdered bile liquid extract of animal origin, combined with 1-200%, preferably 1-100%, more preferably 1-10% by weight of an extract produced from turmeric *(Curcuma spp.)* relative to the weight of the bile extract and 1-100%, preferably 10-50%, more preferably 10-20% by weight of an extract produced from ginger *(Zingiber officinale)* relative to the weight of the bile extract; and optionally, as secondary active agent, 10-20% by weight of papain relative to the weight of the bile extract and/or 5-15% by weight of bromelain relative to the weight of the bile extract and/or 25-75% by weight of α-amylase relative to the weight of the bile extract and/or 25-75% by weight of a lipase of plant origin relative to the weight of the bile extract and/or 70-100% by weight of soy lecithin relative to the weight of the bile extract and/or 20-40% by weight of an extract of milk thistle *(Silybum marianum)* relative to the weight of the bile extract and/or 10-75% by weight of an extract of artichoke *(Cynara scolymus)* relative to the weight of the bile extract and/or 100-150% by weight of an extract of yarrow *(Achillea millefolium)* relative to the weight of the bile extract and/or 5-60% of an extract of peppermint *(Mentha piperita)* relative to the weight of the bile extract and/or 75-120% by weight of an extract of radish *(Raphanus sativus)* relative to the weight of the bile extract and/or 25-40% by weight of an extract of dwarf everlast *(Helichrysum arenarium)* relative to the weight of the bile extract and/or 5-10% by weight of an extract of mallow *(Althea officinalis)* relative to the weight of the bile extract and/or 5-10% by weight of an extract of fennel *(Foeniculum vulgare)* relative to the weight of the bile extract and/or 5-10% by weight of an extract of liquorice *(Glycyrrhiza glabra)* relative to the weight of the bile extract and/or 5-10% by weight of an extract of white horehound *(Marrubium vulgare)* relative to the weight of the bile extract. Furthermore, the compositions of the invention may comprise one or more therapeutically approved and pharmaceutically well known inert additives.

The use of turmeric in cholagogue compositions is supported by the beneficial effects on the liver observed in traditional medicine and backed by the European Food Safety Authority (EFSA). According to the evaluation of EFSA on the effects of nutrients or substances, turmeric prevents the accumulation of fats and facilitates their stockage by the liver and helps maintain the health of the liver. Ginger - besides being a widely used spice - is commonly used by traditional medicine to treat various types of "stomach problems", including motion sickness, morning sickness, colic, upset stomach, gas, diarrhea, nausea caused by cancer treatment, nausea and vomiting after surgery, as well as loss of appetite.

To our best knowledge, the combination of such small amounts of turmeric extract and ginger extract as used in this invention, has never been shown to be effective in suppressing the unpleasant taste and/or odor of compositions comprising bile extract.

Papain is a cysteine protease derived from papaya *(Carica papaya).* Papaya is used to activate digestive enzymes. A food supplement containing protease enzymes is particularly recommended after heavy meals and, as the years pass, to complement the weakening secretion of enzymes that break down proteins.

According to the EFSA, bromelain, an extract derived from pineapple, is a digestive aid (the EFSA accepted number of this "claim on general function" is: 4662) and helps to support digestion (2213). The lipase enzymes of plant origin help to increase bioavailability of nutrients from food by catalyzing the hydrolysis of fats. Alpha-amylase hydrolyses large polysaccharides.

Furthermore, according to the EFSA, soy lecithin is necessary for the metabolism of fat (1597) and contributes to the normal functioning of the liver (1633). The extract of milk thistle helps to maintain the liver function and additionally promote the digestion (3821) and is a protector and regenerator when products that oxidize at hepatic level are administered (4481). Artichoke extract may support a normal secretion and the drainage at the liver and the gall bladder level (4553). Yarrow is traditionally used to facilitate the digestion and contributes to the digestive comfort (3717). Peppermint helps with flatulence and belly spasm (2696) besides supporting digestion (2698). Radish contributes to the elimination function of the gastrointestinal tract and to bile flow function, supports healthy liver activity (2124). The use of the extract of dwarf everlast beneficially affects the function of the liver and gallbladder and may have a beneficial effect in the case of an acute condition of chronic pancreatitis (2450). Mallow is useful in inflammation and irritation of the alimentary canal. Fennel is widely employed as a carminative, both in humans and in veterinary medicine to treat flatulence by encouraging the expulsion of intestinal gas. Liquorice is used for various digestive system complaints including stomach ulcers, colic, and ongoing inflammation of the lining of the stomach (chronic gastritis) while white horehound is used for digestion problems including loss of appetite, indigestion, bloating, gas, diarrhea, constipation, and liver and gallbladder complaints.

The cholagogue compositions of the invention may be produced by the following method variants:
a) a powdered extract is prepared as primary active agent by desiccation from bile liquid of animal origin, and 1-200%, preferably 1-100%, more preferably 1-10% by weight of an extract produced from turmeric *(Curcuma spp.)* relative to the weight of the bile extract and 1-100%, preferably 10-50%, more preferably 10-20% by weight of an extract produced from ginger *(Zingiber officinale)* relative to the weight of the bile extract is added, and optionally, as secondary active agent, 10-20% by weight of papain relative to the weight of the bile extract and/or 5-15% by weight of bromelain relative to the weight of the bile extract and/or 25-75% by weight of α-amylase relative to the weight of the bile extract and/or 25-75% by weight of a lipase of plant origin relative to the weight of the bile extract and/or 70-100% by weight of soy lecithin relative to the weight of the bile extract and/or 20-40% by weight of an extract of milk thistle *(Silybum marianum)* relative to the weight of the bile extract and/or 10-75% by weight of an extract of artichoke *(Cynara scolymus)* relative to the weight of the bile extract and/or 100-150% by weight of an extract of yarrow *(Achillea millefolium)* relative to the weight of the bile extract and/or 5-60% of an extract of peppermint *(Mentha piperita)* relative to the weight of the bile extract and/or 75-120% by weight of an extract of radish *(Raphanus sativus)* relative to the weight of the bile extract and/or 25-40% by weight of an extract of dwarf everlast *(Helichrysum arenarium)* relative to the weight of the bile extract and/or 5-10% by weight of an extract of mallow *(Althea officinalis)* relative to the weight of the bile extract and/or 5-10% by weight of an extract of fennel *(Foeniculum vulgare)* relative to the weight of the bile extract and/or 5-10% by weight of an extract of liquorice *(Glycyrrhiza glabra)* relative to the weight of the bile extract and/or 5-10% by weight of an extract of white horehound *(Marrubium vulgare)* relative to the weight of the bile extract is added, and the obtained mixture is homogenized by known procedures and formulated as a food supplement together with additives commonly used in food supplements; or
b) a bile liquid of animal origin is boiled, 1-200%, preferably 1-100%, more preferably 1-10% by weight of an extract produced from turmeric *(Curcuma spp.)* relative to the weight of the bile extract and 1-100%, preferably 10-50%, more preferably 10-20% by weight of an extract produced from ginger *(Zingiber officinale)* relative to the weight of the bile extract is added, and optionally, 1 to 200 percent by weight of a blend of one or more of the above medicinal herbs relative to the expected dry weight of the bile extract is added to the hot bile liquid, the herb blend is optionally removed after boiling for 5-20 minutes, then the mixture - comprising the bile liquid, the turmeric, the ginger and, optionally, the herb blend or the extract of the herb blend - is evaporated by additional boiling to reach colloidal state, the mixture is cooled down, the obtained solid is disintegrated and formulated as a food supplement together with additives commonly used in such compositions.

A surprisingly significant improvement in acceptability of said previously disclosed cholagogues (Hungarian Patent No. 210 927) was reached when they were formulated together with small amounts of turmeric and ginger extracts.

The improved solid cholagogue compositions of the invention, therefore, comprise:
(a) bile extracts;
(b) 1-200%, preferably 1-100%, more preferably 1-10% by weight of an extract produced from turmeric *(Curcuma spp.)* relative to the weight of the bile extract and 1-100%, preferably 10-50%, more preferably 10-20% by weight of an extract produced from ginger *(Zingiber officinale)* relative to the weight of the bile extract, and
(c) optionally, 10-20% by weight of papain relative to the weight of the bile extract and/or 5-15% by weight of bromelain relative to the weight of the bile extract and/or 25-75% by weight of α-amylase relative to the weight of the bile extract and/or 25-75% by weight of a lipase of plant origin relative to the weight of the bile extract and/or 70-100% by weight of soy lecithin relative to the weight of the bile extract and/or 20-40% by weight of an extract of milk thistle *(Silybum marianum)* relative to the weight of the bile extract and/or 10-75% by weight of an extract of artichoke *(Cynara scolymus)* relative to the weight of the bile extract and/or 100-150% by weight of an extract of yarrow *(Achillea millefolium)* relative to the weight of the bile extract and/or 5-60% of an extract of peppermint *(Mentha piperita)* relative to the weight of the bile extract and/or 75-120% by weight of an extract of radish *(Raphanus sativus)* relative to the weight of the bile extract and/or 25-40% by weight of an extract of dwarf everlast *(Helichrysum arenarium)* relative to the weight of the bile extract and/or 5-10% by weight of an extract of mallow *(Althea officinalis)* relative to the weight of the bile extract and/or 5-10% by weight of an extract of fennel *(Foeniculum vulgare)* relative to the weight of the bile extract and/or 5-10% by weight of an extract of liquorice *(Glycyrrhiza glabra)* relative to the weight of the bile extract and/or 5-10% by weight of an extract of white horehound *(Marrubium vulgare)* relative to the weight of the bile extract.

A preferred composition according to the invention comprises:
(a) bile extracts;
(b) 1-10% by weight of an extract produced from turmeric *(Curcuma spp.)* relative to the weight of the bile extract and 10-20% by weight of an extract produced from ginger *(Zingiber officinale)* relative to the weight of the bile extract.

Another preferred composition according to the invention comprises:
(a) bile extracts;
(b) 1-10% by weight of an extract produced from turmeric *(Curcuma spp.)* relative to the weight of the bile extract and 10-20% by weight of an extract produced from ginger *(Zingiber officinale)* relative to the weight of the bile extract;
(c) 10% of an extract of peppermint *(Mentha piperita)* relative to the weight of the bile extract, 7.5% by weight of an extract of white horehound *(Marrubium vulgare)* relative to the weight of the bile extract, 7.5% by weight of an extract of liquorice *(Glycyrrhiza glabra)* relative to the weight of the bile extract, 7.5% by weight of an extract of mallow *(Althea officinalis)* relative to the weight of the bile extract and 5% by weight of an extract of fennel *(Foeniculum vulgare)* relative to the weight of the bile extract.
Yet another preferred composition according to the invention comprises:
(a) bile extracts;
(b) 1-10% by weight of an extract produced from turmeric *(Curcuma spp.)* relative to the weight of the bile extract and 10-20% by weight of an extract produced from ginger *(Zingiber officinale)* relative to the weight of the bile extract;
(c) 15% by weight of papain relative to the weight of the bile extract, 10% by weight of bromelain relative to the weight of the bile extract, 50% by weight of α-amylase relative to the weight of the bile extract, 50% by weight of a lipase of plant origin relative to the weight of the bile extract, 83.3% by weight of soy lecithin relative to the weight of the bile extract, 31.7% by weight of an extract of milk thistle *(Silybum marianum)* relative to the weight of the bile extract and 50% by weight of an extract of artichoke *(Cynara scolymus)* relative to the weight of the bile extract and 125% by weight of an extract of yarrow *(Achillea millefolium)* relative to the weight of the bile extract.

Yet another preferred composition according to the invention comprises:
(a) bile extracts;
(b) 30.3% by weight of an extract produced from turmeric *(Curcuma spp.)* relative to the weight of the bile extract and 10-20% by weight of an extract produced from ginger *(Zingiber officinale)* relative to the weight of the bile extract;
(c) 40% of an extract of peppermint *(Mentha piperita)* relative to the weight of the bile extract, 100% by weight of an extract of radish *(Raphanus sativus)* relative to the weight of the bile extract, 16% by weight of an extract of artichoke *(Cynara scolymus)* relative to the weight of the bile extract, 33.3% by weight of an extract of dwarf everlast *(Helichrysum arenarium)* relative to the weight of the bile extract, 83.3% by weight of soy lecithin relative to the weight of the bile extract and 31.7% by weight of an extract of milk thistle *(Silybum marianum)* relative to the weight of the bile extract.

The compositions described herein may be administered orally, suitably in the form of tablets, powders, coated tablets, hard or soft gelatin capsules. All dosage forms may be formulated so as to support controlled adsorption of the different components either in the gaster or in the (small) intestine.

The composition according to the invention is preferably administered in the form of enteric film-coated tablets. Overdosing concerning the amount or duration of the composition has no harmful outcome. Suitably 1 or 2 tablets are administered before or after main meals.

In accordance with the above, the invention also concerns the combined use of turmeric and ginger for improving the ingestion and/or long term acceptability of oral cholagogue compositions.

The present invention is illustrated by the following example without limiting it thereto.

### Example 1

Dilute liquid bile is maintained above boiling temperature by heating. During evaporization, after a volume loss of 90 to 95 % by weight, the material solidifies due to cooling.

The resulting base material is finely disintegrated, and a previously prepared medicinal herb blend of the following composition is added thereto in a stirring and homogenizing apparatus:
30.3% by weight of turmeric extract by weight of the bile extract
10% by weight of ginger extract by weight of the bile extract
100% by weight of radish extract by weight of the bile extract
40% by weight of peppermint extract by weight of the bile extract
16% by weight of artichoke extract by weight of the bile extract
33.3% by weight of dwarf everlast extract by weight of the bile extract
83.3% by weight of soy lecithin by weight of the bile extract
31.7% by weight of milk thistle extract by weight of the bile extract.

The novel and better ingestible cholagogue compositions according to the present invention, as exemplified above, are surprisingly more acceptable for individuals in need of such a treatment and provide a widespread ingestion alleviating effect and an improved choleretic effect compared to the corresponding cholagogues described in Hungarian Patent No. 210 927.

## Claims

1. A cholagogue composition comprising
(a) a bile extract of animal origin; in combination with
(b) 1-200% by weight of an extract of turmeric *(Curcuma spp.)* relative to the weight of the bile extract and 1-100% by weight of an extract of ginger *(Zingiber officinale)* relative to the weight of the bile extract.

2. The cholagogue composition according to claim 1, comprising
(a) a bile extract of animal origin; in combination with
(b) 1-100% by weight of an extract of turmeric *(Curcuma spp.)* relative to the weight of the bile extract and 10-50% by weight of an extract of ginger *(Zingiber officinale)* relative to the weight of the bile extract.

3. The cholagogue composition according to claim 1, comprising
(a) a bile extract of animal origin; in combination with
(b) 1-10% by weight of an extract of turmeric *(Curcuma spp.)* relative to the weight of the bile extract and 10-20% by weight of an extract of ginger *(Zingiber officinale)* relative to the weight of the bile extract.

4. The cholagogue composition according to any of claims 1-3, wherein the composition further comprises papain and/or bromelain and/or α-amylase and/or a lipase of plant origin and/or soy lecithin and/or one or more extract(s) of the herb(s) selected from milk thistle *(Silybum marianum),* artichoke *(Cynara scolymus),* yarrow *(Achillea millefolium),* peppermint *(Mentha piperita),* radish *(Raphanus sativus),* dwarf everlast *(Helichrysum arenarium),* mallow *(Althaea officinalis),* fennel *(Foeniculum vulgare),* liquorice *(Glycyrrhiza glabra) and* white horehound *(Marrubium vulgare).*

5. The cholagogue composition according to claim 4, wherein the composition comprises 10-20% by weight of papain relative to the weight of the bile extract and/or 5-15% by weight of bromelain relative to the weight of the bile extract and/or 25-75% by weight of α-amylase relative to the weight of the bile extract and/or 25-75% by weight of a lipase of plant origin relative to the weight of the bile extract and/or 70-100% by weight of soy lecithin relative to the weight of the bile extract and/or 20-40% by weight of an extract of milk thistle *(Silybum marianum)* relative to the weight of the bile extract and/or 10-75% by weight of an extract of artichoke *(Cynara scolymus)* relative to the weight of the bile extract and/or 100-150% by weight of an extract of yarrow *(Achillea millefolium)* relative to the weight of the bile extract and/or 5-60% of an extract of peppermint *(Mentha piperita)* relative to the weight of the bile extract and/or 75-120% by weight of an extract of radish *(Raphanus sativus)* relative to the weight of the bile extract and/or 25-40% by weight of an extract of dwarf everlast *(Helichrysum arenarium)* relative to the weight of the bile extract and/or 5-10% by weight of an extract of mallow *(Althea officinalis)* relative to the weight of the bile extract and/or 5-10% by weight of an extract of fennel *(Foeniculum vulgare)* relative to the weight of the bile extract and/or 5-10% by weight of an extract of liquorice *(Glycyrrhiza glabra)* relative to the weight of the bile extract and/or 5-10% by weight of an extract of white horehound *(Marrubium vulgare)* relative to the weight of the bile extract.

6. The cholagogue composition according to claim 4, wherein the composition comprises 10% of an extract of peppermint *(Mentha piperita)* relative to the weight of the bile extract, 7.5% by weight of an extract of white horehound *(Marrubium vulgare)* relative to the weight of the bile extract, 7.5% by weight of an extract of liquorice *(Glycyrrhiza glabra)* relative to the weight of the bile extract, 7.5% by weight of an extract of mallow *(Althea officinalis)* relative to the weight of the bile extract and 5% by weight of an extract of fennel *(Foeniculum vulgare)* relative to the weight of the bile extract.

7. The cholagogue composition according to claim 4, wherein the composition comprises 15 % by weight of papain relative to the weight of the bile extract, 10% by weight of bromelain relative to the weight of the bile extract, 50% by weight of α-amylase relative to the weight of the bile extract, 50% by weight of a lipase of plant origin relative to the weight of the bile extract, 83.3% by weight of soy lecithin relative to the weight of the bile extract, 31.7% by weight of an extract of milk thistle *(Silybum marianum)* relative to the weight of the bile extract and 50% by weight of an extract of artichoke *(Cynara scolymus)* relative to the weight of the bile extract and 125% by weight of an extract of yarrow *(Achillea millefolium)* relative to the weight of the bile extract.

8. The cholagogue composition according to claim 4, wherein the composition comprises 40% of an extract of peppermint *(Mentha piperita)* relative to the weight of the bile extract, 100% by weight of an extract of radish *(Raphanus sativus)* relative to the weight of the bile extract, 16% by weight of an extract of artichoke *(Cynara scolymus)* relative to the weight of the bile extract, 33.3% by weight of an extract of dwarf everlast *(Helichrysum arenarium)* relative to the weight of the bile extract, 83.3% by weight of soy lecithin relative to the weight of the bile extract and 31.7% by weight of an extract of milk thistle *(Silybum marianum)* relative to the weight of the bile extract.

9. The cholagogue composition according to any preceding claims, for use in the prevention and/or treatment of biliary and/or gastrointestinal discomfort or condition for a prolonged period.

10. Use of turmeric and ginger in combination for improving the ingestion and/or long term acceptability of oral cholagogue compositions.
